# EUROPEAN PATENT SPECIFICATION

(11) **EP 1 019 420 B1**
(45) Date of publication and mention of the grant of the patent: **06.08.2003**
(21) Application number: 96915218.0
(22) Date of filing: 08.05.1996
(51) Int. Cl.: C07F 15/00, G01N 33/58

(54) **METHODS FOR THE PRODUCTION OF PLATINUM-BASED LINKERS BETWEEN LABELS AND BIO-ORGANIC MOLECULES, FOR LABELLING BIO-ORGANIC MOLECULES, FOR DETECTING BIOLOGICAL SUBSTANCES OF INTEREST**
VERFAHREN ZUR HERSTELLUNG VON LINKERN ZWISCHEN MARKIERUNGSGRUPPEN UND BIO-ORGANISCHEN MOLEKÜLEN AUF DER BASIS VON PLATIN FÜR DIE MARKIERUNG VON BIO-ORGANISCHEN MOLEKÜLEN ZUR ENTDECKUNG INTERESSANTER BIOLOGISCHER SUBSTANZEN
PROCEDES DE PRODUCTION DE SEGMENTS DE LIAISON A BASE DE PLATINE ENTRE DES MARQUEURS ET DES MOLECULES BIO-ORGANIQUES, POUR LE MARQUAGE DE MOLECULES BIO-ORGANIQUES, LA DETECTION DE SUBSTANCES BIOLOGIQUES A ETUDIER

(30) Priority: 09.05.1995 EP 95201197
(43) Date of publication of application: 19.07.2000
(73) Proprietor: Kreatech Biotechnology B.V., 1032 LG Amsterdam (NL)
(72) Inventor: HOUTHOFF, Hendrik, Jan, NL-1023 NB Amsterdam (NL); REEDIJK, Jan, NL-2334 CD Leiden (NL); JELSMA, Tinka, NL-1326 DG Almere (NL); VAN ES, Remco, Maria, NL-1541 NR Koog a/d Zaan (NL); VAN DEN BERG, Franciscus, Michiel, NL-2134 JB Hoofddorp (NL); LEMPERS, Edwin, Leo, Mario, NL-1788 GB Julianadorp (NL); BLOEMINK, Marieke, Johanna, NL-2341 LM Oegstgeest (NL)
(74) Representative: Smulders, Theodorus A.H.J., Ir.
(86) International application number: NL9600198
(87) International publication number: WO96035696

(56) References cited:
- EP-A- 0 282 672
- EP-A- 0 386 243
- WO-A-92/01699
- GB-A- 2 148 891
- US-A- 4 207 416
- PATENT ABSTRACTS OF JAPAN vol. 5, no. 180 (C-79) [852] , 19 November 1981 & JP,A,56 103192 (YOSHINORI KITANI), 18 August 1981,

## Description

The invention relates to methods for the production of linkers (linking moieties between labels and bio-organic molecules, which linkers are based on platinum compounds. Platinum (coordination) compounds have been considered interesting molecules for a very long time. For a review of these compounds and their uses we refer to Reedijk et al. (Structure and Bonding 67, p.53-89, 1987). Especially Cis-platinum has received a lot of attention as a possible anti-tumour drug. This anti-tumour reactivity of platinum compounds originates from their having at least two reactive groups (preferably cis-oriented towards each other), which make it possible to cross-link DNA molecules, thereby inhibiting the replication of these DNA molecules.

A different use of platinum (coordination) compounds has been disclosed in PCT application (WO92/01699) wherein a platinum compound having two reactive moieties (denominated as leaving groups therein) is reacted with a fluorescein to obtain a labelled platinum compound having only one reactive moiety which can bind (non-covalently) to a nucleic acid, preferably at the N-7 position of a guanine residue. The present invention provides improved methods of preparing such platinum compounds which are suitable for use as linkers in such labelling substances, improved methods of preparing labelled substances and the use of such substances in preparing diagnostic test kits.

In WO92/01699 the starting compounds disclosed are platinum(II)(ethylenediamine)dichloride or [platinum(II)(ethylenediamine)(Me₂SO)Cl]⁺Cl⁻.

Both compounds have certain disadvantages for use as a linker. The first one can be obtained commercially, the second one (the preferred one) must be synthesized as the chloride salt. In the dichloride compound, the Cl⁻ions are less readily substituted by a marker group or a guanine group, respectively. Moreover, since the two Cl⁻ ions have about the same reactivity, it happens that both ions are substituted by a label, leaving no reactive moiety for coupling the labelled substance to a bio-molecule of interest. It is for that reason that compounds having different reactive moieties are very much preferred for use in labelling. This is true for the second compound, however, the total DNA labelling for this compounds will be appreciably longer, up to several hours in stead of several minutes, making the product less suitable for routine use.

We have now found a very simple and reliable method to produce symmetrical linkers including the first one just mentioned and a number of novel ones, through selection of suitable starting compounds of the formula PtE₄ wherein E is an electronegative group, preferably a halogen or NO₃⁻ or SO₃⁻. The reaction, which is described in the examples, of these starting compounds with ethylenediamine is very simple and efficient. Moreover, this reaction leads to very suitable symmetric intermediate compounds for producing labelled substances. The advantages of working with these starting compounds for producing labelled substances have never been disclosed and these starting compounds have never been used for this purpose. A major advantage of using these compounds is that when a stabilizing bridge for the resulting platinum compound has to be attached that no blocking reagents have to be employed. Another advantage is that the resulting intermediate compounds can again be labelled without the use of blocking agents. Therefor steps removing blocking agents can be eliminated completely. Furthermore the yields of these reactions are very high. Another advantage of the use of these symmetrical starting compounds is that no mixtures of different resulting compounds can be formed, which may interfere with the following reaction and reduce yield or require extra separation steps. A very suitable intermediate compound according to the invention is platinum(II)(ethylenediamine)(NO₃)₂. Surprisingly, hardly any double labelling occurs with these compounds, which is in contrast with the dichloride compound of WO92/01699. This substance can very easily be provided with a suitable labelling group, resulting in a labelling substance which can still, through substitution of the remaining NO₃ group be linked to a bio-organic molecule to be labelled or detected, which reaction proceeds much faster than with the known platinum compounds. Furthermore the methods for producing this compound and the resulting compound do not involve highly toxic substances such as DMSO.

The intermediate compounds can be labelled with any suitable label (also known as marker). These labels may be radioactive labels, enzymes (which need reaction with a substrate to be detected), specific binding pairs components such as avidin, streptavidin or biotin, biocytin, iminobiotin, colloidal dye substances, fluorochromes (rhodamin, etc.), reducing substances (eosin, erythrosin, etc.), (coloured) latex sols, digoxigenin, metal sols or other particulate sols (selenium, carbon and the like), dansyl lysin, antibodies, protein A, protein G, etc. These labels may be attached to the linkers directly or through spacer arms (preferably polylysin). Because the labelling of nucleic acids and proteins is so easy, it is possible to sell the linker-label compounds separately in a kit so that any user can produce the labelled substances easily, or can attach by the same procedure many different labels of choice to the substances. This allows for multiple detections in one assay. Furthermore the known advantages of the platinum labelling compounds (from WO92/01699 for instance) are of course also obtained with the present methods and compounds. Another advantage of the platinum compounds is that they can be detected more or less directly by using the platinum as a nucleus for depositing silver or other metal crystals.

Almost every bio-organic molecule which contains an accessible S (sulphur) or an N (nitrogen) can be labelled with the platinum compounds. A very suitable bio-organic molecule to be labelled with the labelling substance according to the invention is nucleic acid (nucleotides, oligonucleotides, DNA, RNA, homo duplexes, heteroduplexes, or multiplexes). The platinum binds very easily (non-covalently) to the N-7 position of guanine residues. This way DNA or RNA molecules, be it single stranded or otherwise can be easily detected, but it also allows for the production of probes for hybridization techniques wherein unlabelled DNA/RNA molecules hybridize to the labelled probe. The platinum compounds do hardly interfere with the hybridization, if at all. Also, this technique obviates the use of modified nucleotides in preparing probes. However, proteins, peptides and other bio-organic molecules can also be labelled with the labelling substances according to the invention.

The platinum compounds according to the invention are also very suitable for attaching bio-organic molecules to solid surfaces such as nitrocellulose, nylon filters or microtiter plates.

Nucleotides modified in accordance with the practices of this invention and oligo- and polynucleotides into which the modified nucleotides have been incorporated or oligo- and polynucleotides that have been directly modified using these novel platinum compounds may be used as probes in biomedical research, clinical diagnostics and recombinant DNA technology.

The wide variety of utilities are based upon the ability of the platinum compounds to form stable complexes with polypeptides which in turn can be detected either by means of detectable moieties which are attached to or which interact with the polypeptide. Some uses include detecting and identifying nucleic acid-containing etiological agents, e.g. bacteria and viruses; screening bacteria for antibiotic resistance; screening animals for genetic disorders in relation to pharmaceutical effects; diagnosing genetic disorders, e.g. trisomy 21, sickle cell anemia: chromosomal karyotyping: and identifying tumour cells.

Other -advantages and embodiments of the invention will become clear from the following experimental part.

### EXPERIMENTAL

### SYNTHESIS OF INTERMEDIATE PLATINUM COMPOUNDS

These compounds may be prepared by a process which involves:
(a) reacting a compound in which the anion has the structure: 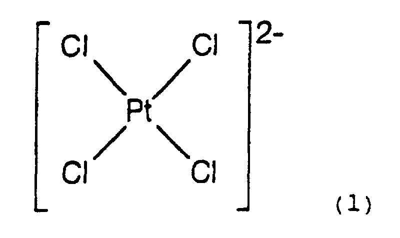 with KI in a suitable solvent under suitable conditions so as to form a iodated platinum compound wherein the anion has the structure: 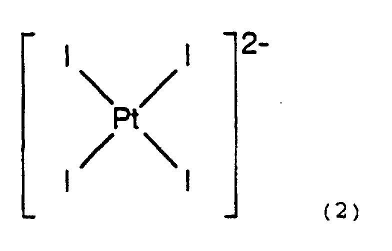
(b) reacting said iodated platinum compound (2) with ethylenediamine in a suitable solvent so as to form a diethyleneamine iodated platinum compound as represented by the formula Pt(en)I₂ and which has the structure: 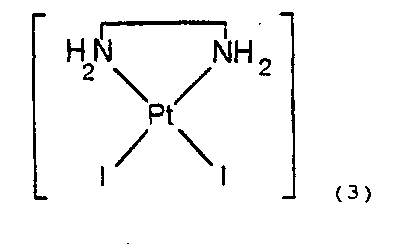
(c) reacting said compound (3) with AgNO₃, the reaction being carried out in a suitable solvent, under suitable conditions so as to form a compound having the structure: 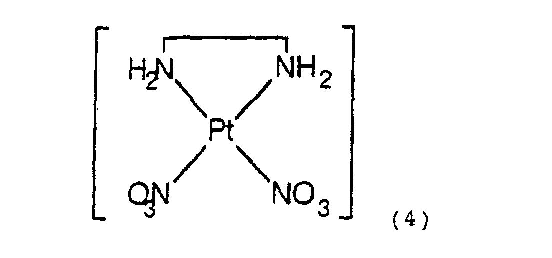
(d) reacting said compound (4) with KCl in a suitable solvent under suitable conditions so as to form a compound having the structure: 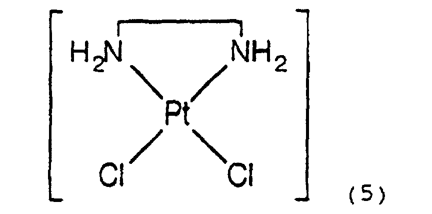
(e) reacting said compound (5) with AgNO₃ in a suitable solvent, under suitable conditions so as to form a compound having the structure: 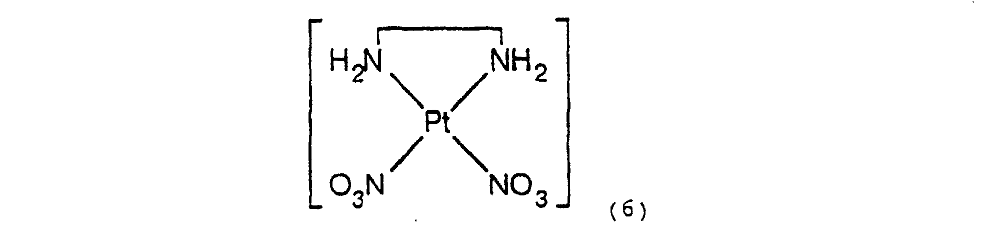
(f) recovering said compound (6) as modified platinum starting compound for the synthesis of hapten-bound Pt(en) compounds for use as DNA and/or RNA label.

Compounds (4) and (6) are of course the same, both reactions preparing them have been given as alternatives.

### Example 1

### Preparation of Pt(en)-diamine starting material.

### Preparation of Platinumethylenediamine(NO₃)₂: starting material.

### Pt(en)(NO₃)₂

### All reactions are performed in the dark.

Dissolve 1 gram Potassium tetrachloroplatinate(II), K₂PtCl₄ (2.4 mmol, Sigma) in 50 ml millipore (filtered water) and stir at room temperature. Add 10 equivalents of Potassium iodide, Kl (24 mmol, 3.999 g, Sigma). The colour of the solution will immediately turn from orange into dark red (K₂PtI₄), stir for 5 minutes.

Add one equivalent ethylenediamine (2.4 mmol, 160.8743 µl, Merck 11=0.9 kg) after diluting 161 µl ethylenediamine in 5 ml millipore very slowly to the platinum solution, mix this solution for 1 hour at room temperature.

A yellow/brown precipitate, Pt(en)I₂, will be formed and the liquid standing above should be clear.

Filter the solution through a 1.0 µm membrane filter (Schleicher&Schuell), wash the precipitate with millipore, ethanol and diethylether (in this order). Dry the Pt(en)I₂ for at least 4 hours in a vacuum dry-oven at 37°C.

Weigh the dried Pt(en)I₂ (-1.07 g) and suspend it in 45 ml millipore/5 ml aceton, the solution will be cloudy. Add 1.95 equivalent of AgNO₃ (M = 169.9, Sigma). Stir the reaction overnight at room temperature.

Filter the solution through a 1.0 µm membrane filter, the precipitate is Silver iodide, AgI, the filtrate should be clear.

Add to 0.5 ml of the filtrate, containing Pt(en)(NO₃)₂, an excess of KCl or NaCl and make sure that no white precipitate is formed immediately after adding the excess of KCl or NaCl. If no white precipitate (only a yellow one) is formed then add an excess of KC1 or NaCl to the entire filtrate. After the yellow precipitate is formed filter the solution and wash the precipitate. (Pt(en)Cl₂) with millipore, ethanol and diether.

Dry the precipitate for at least 4 hours in a vacuum dryoven at 37°C.

Weigh the dry Pt(en)Cl₂ (M=326,1), and suspend it in 45 ml millipore/5 ml aceton and stir the cloudy suspension. Add 1.95 equivalent AgNO₃ and stir the solution overnight at room temperature. The colour of the solution will become white, due to the formation of AgCl.

Filter the solution in the dark and evaporate the filtrate to remove the aceton by rotation evaporation untill 25 ml of the filtrate is left. The filtrate is then freezedried. The product is checked by NMR or Infrared Absorption Spectroscopy. An element analysis is given below.

**Table I**

| Pt(en)(NO₃)₂ M_{w} = 379.1 20/1 batch | | | | |
|---|---|---|---|---|
| | A | B | Δ A-B | Theor. |
| Sample 2 | 5.79 % C | 5.15 % C | -0.86 % | 6.33 % |
| | 2.03 % H | 1.81 % H | +0.19 % | 2.11 % |
| | 12.1 % N | 13.3 % N | -2.07 % | 14.77 % |
| | 48.2 % Pt | 48.4 % Pt | -3.16 % | 51.46 % |
| | 23.5 % O | 23.1 % O | -2.02 % | 25.32 % |
| | <0.2 % Na | | | |

There was not sufficient material for Cl-test.
V₂O₅ was added for the C,H-, N- and S-combustions.

**Table II**

| Compound ULS 95 RM005 | Reqd. | Theory | Found (A)% | Ref. | Found (B)% | Ref. |
|---|---|---|---|---|---|---|
| Probable | C | 6.3 | 6.47 | F2576 | | |
| Structure | H | 2.1 | 2.11 | " | | |
| [Pt(en)(NO₃)₂ | N | 14.8 | 14.48 | " | | |
| | O | | | | | |
| | S | | | | | |
| | Hal | | | | | |
| | | | | | | |
| | Metal | | | | | |

### Example-2

### Preparation of immunologically detectable hapten-Pt(en)compounds

### Preparation of platinum ethylene diamine-ε-(6-(Biotinoyl)amino) hexanoyl-L-lysine-nitrate

### [Pt(en)(Biocytin-X)(NO₃)]HO₃.

### All reactions are performed in the dark.

Dissolve 31.6 mg biocytin-X (0.065 mmol, Molecular Probes, Inc, USA) in 15 ml millipore in the dark and heat the solution slightly (up to a maximum 40°C) untill dissolving and adjust the pH to 7-8 with 0.4 M NaOH. Dissolve 23.4 mg Pt(en)(NO₃)₂ (M=379.1, 0.062 mmol)(see example 1) in 10 ml millipore in the dark and heat the solution slightly (up to a maximum of 50°C) until Pt(en)(NO₃)₂ is completely dissolved.

When both reagents are completely dissolved add the biocytin solution to the platinum solution and let them react for 210 minutes at room temperature in the dark. The pH of the solution has to be 7.0, so the pH is monitored during the reaction and either H₂O or HNO₃ is added if necessary.

Isolate the product [Pt(en)(biocytin-X)(NO₃)]NO₃ by means of freeze drying.

Stability of the product is ensured by the binding of Pt not only to the amino group of biocytin-X, but also to the carboxylate group of biocytin-X. Due to the latter binding kinetics of the platinum compound a chelate ring form is created which will account for the stability of the product once it is dissolved in water, because no polymerisation reactions can occur. An element analysis of [Pt(en)(biocytin-X)(NO₃₎]NO₃ is given below.

**Table III**

| BIO-ULS [Pt(en)BIO]NO₃ M_{w} = 689,6 | | | | |
|---|---|---|---|---|
| | A | B | Δ A-B | Theor. |
| Sample 1 | 31.49 % C | 31.42 % C | -0.064 % | 31.50 % |
| | 5.63 % H | 5.40 % H | +0.374 % | 5.89 % |
| | 13.2 % N | 13.0 % N | -1.24 % | 14.34 % |
| | 18.3 % Pt | 18.5 % Pt | - 9.95 % | 28.35 % |
| | 3.65 % S | 3.67 % S | -1.0 % | 4.66 % |
| | 19.2 % O | 19.0 % O | -2.826 % | 21.926 % |
| | 3.23 % Na | | | |

There was not sufficient material for Cl-test.

### Example 3

### Preparation of Platinum ethylenediamine-Digoxigenin^{R}-L-lysin-nitrate.

### [Pt(en)(Digoxigenin)(NO₃)] (NO₃).

Dissolve Pt(en)(NO3)2 ( 0.062 mmol, 23,65 mg) in 20 ml methanol and heat to 50°C until Pt(en)(NO₃)₂ is completely dissolved.

Dissolve digoxigenin-L-lysin (45,3 mg) in 20 ml methanol, heat until all solid material is dissolved.

Add the two solutions together and react for at least 3 hours. Isolate the end product by rotation evaporation. Digoxigenin^{R} is a registered trademark of Boehringer Mannheim.

### Example 4

### Preparation of fluorescent Platinum(en) compounds

### Preparation of Platinum ethylenediamine-Fluoresceinamine-nitrate.

### [Pt(en)(Fluoresceinamine)(NO₃)]NO₃.

### All reactions are performed in the dark.

Dissolve Pt(en)(NO3)2 (0.25 mmol, 94,8 mg) in 10 ml methanol/2 ml water, by heating until dissolving.

Dissolve fluoresceinamine (0.27 mmol, 95,5 mg, Sigma) in 10 ml methanol.

Add the fluoresceinamine solution to the platinum solution, an immediate change of colour is observed.

Run the reaction for at least 3 hours.

Isolate the crystalline end product by rotation evaporation, followed by freeze drying.

### Example 5

### Preparation of Platinum ethylendiamine-5'acetamidoFluorescein-nitrate.

### [Pt(en)(5'acetamidoFluorescein)(NO₃)]NO₃.

### All reactions are performed in the dark.

Dissolve Pt(en)(NO3)2 ( 0.047 mmol, 17.8 mg) in 5 ml methanol, heat until dissolving. Dissolve 5' acetamidofluorescein (0.049 mmol, 20 mg, Molecular Probes Inc.) in 10 ml methanol.

Add the acetamidofluorescein solution to the platinum solution and react for at least 3 hours

Isolate end product by rotation evaporation.

### Example 6

### Preparation of Platinum ethylenediamine ε-(6-(fluorescein-5-carboxamido)hexanoyl)-L-lysine-nitrate.

### [Pt(en) Fluoresceinlysine (NO₃) lNO₃.

### All reactions are performed in the dark.

Dissolve Pt(en)(NO3)2 (0.077 mmol, 29,17 mg) in 10 ml methanol, heat upon dissolving.

Dissolve ε-(6-(fluorescein-5-carboxamido)hexanoyl)-L-lysin (0.081 mmol, 50 mg) in 10 ml methanol.

Add the fluorescein solution to the Platinum solution and react for at least 3 hours. Isolate the end product by rotation evaporation.

### Example 7

### Preparation of Platinum ethylenediamine-5-(and 6)-((N-(5-aminopentyl) amino) carbonyl) tetramethylrhodaminenitrate.

### [Pt(en)(tetramethylrhodamine cadaverine)(NO₃)]NO₃

### All reactions are perfromed in the dark.

Dissolve Pt(en)(NO3)2 (0.074 mmol, 28 mg) in 10 ml methanol, heat upon dissolving.

Dissolve tetramethylrhodamine cadaverine (0.077 mmol, 40 mg, Molecular Probes Inc.) in 10 ml methanol.

Add the two solutions together and react for at least 3 hours.

Isolate the end product by rotation evaporation.

### Example 8

### Preparation of Platinum ethylenediamine-Cascade BlueR cadaverine-nitrate.

### [Pt(en)(Cascade Blue cadaverine)(NO₃)]NO₃.

Dissolve Pt(en)(NO₃)₂ (0.057 mmol, 21,6 mg) in 10 ml demineralised water and heat to 50°C until Pt(en)(NO₃)₂ is completely dissolved.

Dissolve Cascade Blue cadaverine (0.06 mmol, 40 mg., Molecular Probes Inc.) in 10 ml demineralised water.

Add the two solutions together and react for at least 3 hours.

Isolate the end product by freeze-drying.

Cascade Blue^{R} is a registered trademark of Molecular Probes Inc.

### Example 9

### Preparation of

### Platinum ethylenediamine-4,4-difluoro-5,7-dimethyl-4-boraO3a,4a-diaza-s-indacene-3-proprionylethylenediamine-nitrate.

### [Pt(en)(BODIPYR530/550 C3EDA)(NO₃)]NO₃.

Dissolve Pt(en)(NO₃)₂ (0.052 mmol, 19.66 mg) in 10 ml methanol and heat until dissolving .

Dissolve BODIPY 530/550 C3EDA (0.0545 mmol, 256 mg, Molecular Probes Inc.) in 10 ml methanol.
Add the two solutions together and react for at least 3 hours.

Isolate the end product by rotation evaporation.

BODIPY® is a registered trademark of Molecular Probes Inc.

### Example 10

### Preparation of Platinum ethylenediamine-N-ε-(5-dimethylaminonaphtalene-1-sulfonyl)-L-lysin-nitrate [Pt(en)(Dansyl lysin)(NO₃)]NO₃.

Dissolve Pt(en)(NO3)2 (0.125 mmol, 47.5 mg) in 20 ml demineralised water and heat to 50°C until Pt(en)(NO₃)₂ is completely dissolved.

Dissolve Dansyl lysin (0.13 mmol, 50 mg, Molecular Probes Inc.) in 20 ml demineralised water, adjust the pH to 7 - 8 with 0.4 M NaOH.

Add the two solutions together and react for at least 3 hours. Isolate the end product by freezedrying.

### Example 11

### Reaction for coupling Pt(en) - compounds to DNA

### Typical reaction for labelling DNA molecules with a Pt(en)-compound.

5 µg of double stranded DNA is sonicated or DNase treated to yield fragments of 300-500 bp. 6 µg of Pt(en)-compound is added and the volume is adjusted to 50 µl with demineralised water. The reaction mixture is incubated at 65°C for 1 hour. Non-bound Pt(en)-compound is blocked by adding 100 µl of a NADDTC solution. The Pt(en)-compound labelled DNA is purified on a sephadex G-50 column. Readily labelled and purified DNA is stored at -20°C or used directly in a DNA probe based assay. Pt(en)-compound labelled DNA probes can be stored at least 2 years at 20°C without loss of activity and/or specificity.

All applications mentioned are carried out with probes labelled according to this protocol.

### Example 12

### Preparation of Platinum ethylendiamine-rhodamine 123-nitrate.

### [Pt(en)(rhodamine 123) (NO₃)]NO₃.

### All reactions are performed in the dark.

Dissolve Pt(en)(NO₃)₂ (0.058 mmol, 21,9 mg) in 10 ml N,N-dimethylformamide, heat upon dissolving.

Dissolve rhodamine 123 (0.060 mmol, 22,8 mg) in 8 ml N,N-dimethylformamide.Add the rhodamine solution to the platinum solution and react for at least 3 hours. Isolate the product by rotation evaporation.

### Example 13

### Preparation of Platinum ethylenediamine-(7-amino-4-methylcoumarine)-nitrate.

### [Pt(en)(7-amino-4-methylcoumarine)(NO₃)]NO₃.

### All reactions are performed in the dark.

Dissolve Pt(en)(NO₃)₂ (0.098 mmol, 37,0 mg) in 25 ml methanol/2 ml demineralized water, heat upon dissolving. Dissolve 7-amino-4-methylcoumarine (0.10 mmol, 18 mg) in 10 ml methanol.

Add the 7-amino-4-methylcoumarine solution to the platinum solution and react for at least 4 hours. Isolate the product by rotation evaporation, followed by freezedrying.

### Example 14

### Preparation of Platinum ethylenediamine-5-aminoeosinnitrate.

### [Pt(en)(5-aminoeosin)(NO₃)]NO₃.

### All reactions are performed in the dark.

Dissolve Pt(en)(NO₃)₂ (0.068 mmol, 25.6 mg) in 15 ml N,N-dimethylformamide, heat upon dissolving.

Dissolve 5-aminoeosine (0.075 mmol, 50 mg) in 10 ml N,N-dimethylformamide.

Add the 5-aminoeosine solution to the platinum solution and react for at least 4 hours.

Isolate the product by rotation evaporation.

5-aminoeosin: commercially available by Mol. Probes (A-117)

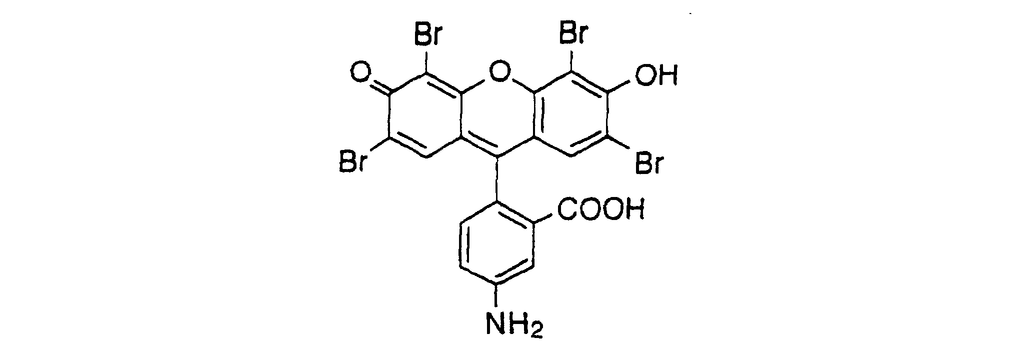

### Example 15

### Preparation of Platinum ethylenediamine-IRD-nitrate.

### [Pt(en)(IRD)(NO₃)]NO₃.

### All reactions are performed in the dark.

Dissolve Pt(en)(NO₃)₂ (0.008 mmol, 3.2 mg) in 5 ml

N,N-dimethylformamide, heat upon dissolving.

Dissolve IRD (presented below) (0.009 mmol, 7.5 mg) in 5 ml N,N-dimethylformamide.

Add the IRD solution to the platinum solution and react for at least 3 hours. Isolate the product by rotation evaporation.

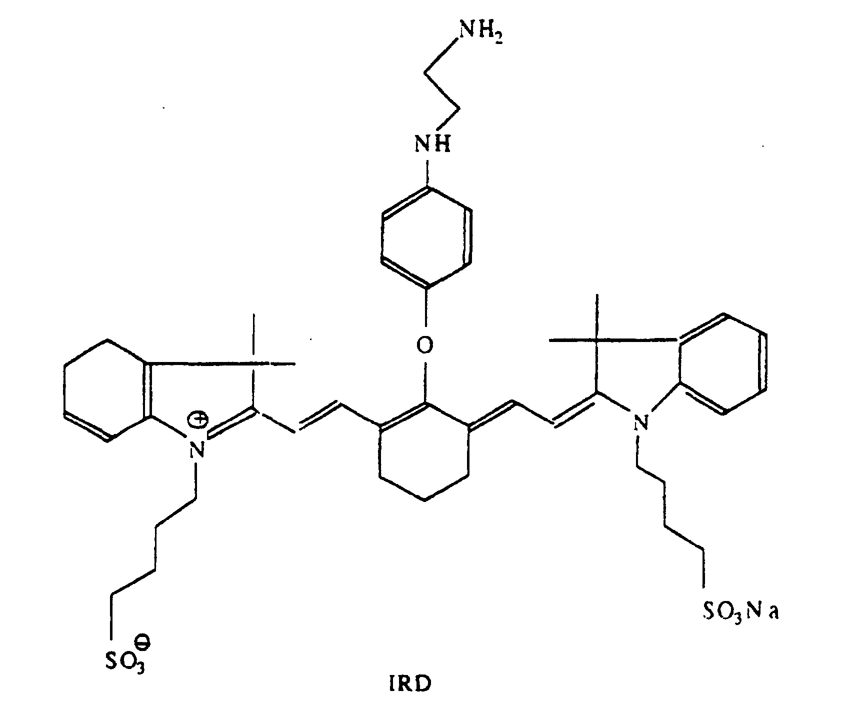

### Example 16

### BIOTIN LABELLING OF DNA PROBES WITH THE UNIVERSAL LINKAGE SYSTEM

### Introduction

The labelling method has been used to label DNA probes with biotin for In Situ Hybridization (ISH). In this example labelling procedures including the protocols and data for quality control procedures, are presented. For biotin labelling a plasmid cloned total DNA of Human Papilloma Virus type 6 (HPV-6, 40% GC basepairs) was used.

### Experimental procedures

### Plasmid DNA preparation

Total DNA of Human Papilloma Virus type 6 was cloned into vector pSp-64. Plasmid DNA was transfected into E.coli (C-600) and plated onto ampicillin containing LB plates. Single colonies were grown overnight in large culture. Plasmid DNA was isolated according to the method of Birnboim and Doly¹, purified by Sepharose C1-2B columnchromatografy (Pharmacia) and checked for inserts by restriction-enzyme-analyses. Plasmid DNA concentration was determined by A260/280 absorbtion. After ethanol precipitation the DNA was reconstituted in 10mM TRIS/HCl pH 7.2, 0.3mM EDTA to a final concentration of 1 µg/µl (batch# 150894) Subsequently this DNA was sonicated (Soniprep 150., MSE) for 3 times 10 minutes (amplitude 5 microns)on ice.

The size of the resulting DNA fragments was determined by 2% agarose gel electrophoresis and found to be in between 200-400 basepairs (batch# 051094).

### Plasmid DNA labelling and purification

Plasmid HPV-6 DNA was labelled with Biotin∼ULS by mixing the following reagents:

| | |
|---|---|
| plasmid HPV-6 DNA (batch# 051094) | 5 µl (1 µg/µl) |
| Biotin-ULS labelling reagent (batch# BX940830) | 8 µl (1 µg/µl) |
| Demineralised water (< 0.2/ µS/cm) | 37 µl |

The 50 µl reaction mixture was incubated for 15 minutes at 85°C.

Excess of labelling reagent was captured by adding 50 µl sodium diethyldithiocarbamate (2% solution in demineralised water) and incubating for 30 minutes at roomtemperature.

Unbound Biotin∼ULS was removed, using a S300 HR microspin column (Pharmacia), by size exclusion chromatography.

Eluent volume was adapted to 500 µl giving a 10 ng/µl biotin HPV-6 probe concentration (batch-06109-4.).

### Quality Control for detection limits

The detection limit of the Biotin probe was determined by direct spot-blot and reversed filterhybridization according to the following protocols:

### Direct spot blot

Biotin labeled HPV-6 probe (batch 061094) was 10 fold serially diluted into spot buffer comprising 900mM sodiumchloride, 90mM sodiumcitrate and 200 µg/ml single stranded salmon sperm DNA giving a dilution series varying from 1000 - 0.1 pg biotin probe per µl.

1 µl spots were applied onto Nitrocellulose membrane and incubated for 2 hours at 80°C to bind the DNA. The biotin probe was visualized using a streptavidin-alkaline phosphatase conjugate (Sigma) combined with an NBT/BCIP precipitating substrate solution (Sigma) according to the following protocol:
- Membranes were soaked in TBS solution containing 0.5% tween20 (TBST) for 5 minutes.
- Membranes were incubated with Strep-AP (3 DEA U/ml) in TBST for 15 minutes at 37°C.
- NC-membranes were washed 3 times 5 minutes in TBS solution followed by a 5 minutes wash step in demineralized water.
- Membranes were incubated with NBT/BCIP substrate solution for 15 minutes at 37°C, subsequently washed in demineralized water and air dried.

### Results

By using this method the detection limit of the biotin DNA probe was found to be less than 1 pg.

### Reversed filterhybridization

HPV-6 DNA (batch 051094) was 1 in 10 diluted in 0.1N NaOH, incubated at 100°C for 5 minutes and directly placed on ice for 5 minutes to make the DNA single stranded.
A 10 fold serial dilution was made in cold 0.1N NaOH to give a serie varying from 10,000 - 1 pg DNA per µl. 1 µl spots were applied onto Nylon membrane (Boehringer Mannheim) and air dried.

Biotin labelled HPV-6 DNA probe was diluted in 5xSSPE 0.5% SDS solution to yield a concentration of 200 ng/ml. This solution was incubated for 5 minutes at 100°C and placed directly on ice for 5 minutes.

Nylon membranes containing target DNA were soaked in 2x SSC for 5 minutes and subsequently incubated with the single stranded probe solution for 2 hours at 37°C. Excess of the biotin probe was removed by three changes in 2x SSPE 0.1% SDS for 10 minutes at 37°C followed by a 5 minutes TBST incubation.

The Biotin probe was visualized by performing the same protocol as described in the direct spotblot method.

### Results

By using this procedure the detection limit of the biotin probe was found to be less than 10 pg.

### Performance in In Situ Hybridisation

The test material consisted of 6 µm paraffin sections of a HPV-6 positive cervical condyloma mounted on organosilane coated glass slides.
The following protocol was applied (unless otherwise stated steps are at roomtemperature):
1 Paraffin sections were dewaxed in 3 changqes of xylene and hydrated in graded ethanol.
2 Sections were rinsed in TBST for 5 minutes.
3 Sections were digested in 0.25% pepsin in 0.1N HCl for 30 minutes at 37°C, dehydrated in graded ethanol and air dried.
4 10 µl of probe solution was applied to a section and covered with a coverslip.
   Probe solution consisted of biotin HPV-6 probe DNA (batch# 061094) in a concentration of 2 ng/µl dissolved in hybridization mixture comprising 0.6M NaCl, 0.06M Na-citrate, 35% formamid,
   10% dextransulphate, 2.5x Denhardts and 10 µl/ml single stranded salmon sperm DNA.
5 Slides were placed on a hotplate set at 95°C for 5 minutes to denature probe and target DNA simultaneously.
6 Hybridisation was performed by placing the slides in a humidified chamber at 37°C for 2 hours.
7 Coverslips were removed and slides were washed in 3 changes of 15mM NaCl, 1.5mM Na-citrate and 5% formamid for 10 minutes at 37°C.
8 Slides were rinsed in TBST.
9 Sections were incubated with Streptavidin AP conjugate (3 DEA U/ml in TBST) for 15 minutes at 37°C.
10 Slides were washed in TBST (3x) and demineralised water (1x) for 5 minutes.
11 Sections were incubated with NBT/BCIP substrate solution for 15 minutes at 37°C.
12 Slides were washed in demineralised water (3x) for 1 minute and sections were mounted in glycerol/gelatin.

### Results

By using the sections showed blue/purple precipitates at the sites of HPV-6 infected cells and minor background in the remaining tissue.

### Conclusions

The results demonstrate that DNA labelled with the ULS method have good label detection limits. The ULS method is very well suited for research, routine and for industrial production of labelled nucleic acids, as the method is fast and easy to perform, very sensitive, and does not include any enzymatic step, which makes it highly reproducible and fitted for an overall low cost production. The ULS method offer a useful alternative equaling conventional non-isotopic labelling methods.

### General references

1. Maniatis T., Sambrook J., Fritsch E.F., Molecular Cloning, Second Edition, Cold Spring Harbor Laboratory Press, ISBN 0-87969-309-6.
2. Keller G.H., Manak M.M., DNA probes, Stockton Press, ISBN 0-333-47659-X.

### Applications

### 1. The use of Pt-DNA linkers in the so called LIDIA technique: Linked DNA immuno Assay.

The LIDIA technique enables the quantitative analysis of small amounts of DNA (or RNA) e.g. after a PCR amplification of the starting material. The technique is sensitive and specific, due to the use of specific DNA(RNA) probes and easy to perform, because of the quick DNA(RNA) Pt-labelling steps.

### Description of the technique:

The technique uses fast Pt labelling compounds to label DNA(RNA) probes

This technique is possible with 3 different approaches.
1. Linking DNA probes molecules to a surface by using a Pt compound which cross-link DNA molecules irreversibly to plastic, nylon or nitrocellulose. Detection of DNA targets can then be accomplished by using classically labelled DNA/RNA probes.(nick translation or chemical modification, random priming)
2. Linking a detectable group to the DNA, to render a DNA molecule into a so-called DNA probe. Binding of DNA compounds to a surface can then be accomplished by using classic techniques known to science (covalent linking to specially treated microtiter plates, baking of DNA molecules onto nitrocellulose or binding of DNA molecules to nylon membranes.
3. A combination of techniques 1 and 2

### Approach 1

An immobilized DNA probe can be used to catch specific target molecules in a sample by using a hybridization technique. Detection of formed hybrids can be done by using different techniques, e.g. a second labelled DNA probe can be used to hybridize with a different site on the target DNA molecule to form a sandwich hybrid. The label can then be detected by using state of the art immunological detection and colouring techniques.

### Approach 2

A volume containing (amplified) detectable DNA(RNA) is directly labelled according to the protocol.

Excess label is quenched by adding NaDDTC or Thioureum. This approach distinguishes itself from other techniques by the fact that the target molecule is labelled in contrast to other assay were labelled DNA(RNA) probes are used to detect the target. The quick binding capacity of the Pt-label compound(ULS) enables a DNA binding step as a routine step in a diagnostic test procedure (normal binding times are 60 minutes at 65°C).

A second step is performed in a microtiter plate precoated with a target specific probe. Incubation is allowed to the formation of stable "Labelled target" and probe hybrids. The direct labelling of target molecules enables the omission of laborious double hybridization techniques where one probe is used to catch the target and another labelled probe is used to detect the immobilized target.

In this method the probes are covalently linked to the microtiter plate to the surface of the wells. The second incubation step has the character of a liquid hybridisation and therefore can be performed very rapidly. This is one of the main innovative features of this approach to quantitive DNA hybridisation techniques.

### Approach 3

Both for the immobilization of DNA probes or DNA targets and for the labelling of DNA probes and targets the newly developed Pt Universal Labelling System can be used. These two DNA linking techniques can be combined into one assay where both the "catcher" and the "detector" are linked to a second substance (either a detectable grouplike biotin, digoxigenin or a carrier surfacelike a plastic stick, microtiter plate or a membrane).

Examples of the technique: the detection of STD related microorganisms in human diagnostics (Chlamydia, Syfilis, AIDS, Herpes, Gonorrhoea, Hep. B,)

### 2. The use of Pt-DNA labels in combination with test strip procedures and formats.

### The "DNA Dipstick".

The DNA dipstick technique enables the qualitative and semi-quantitative analysis of small amounts of DNA(or RNA) e.g. after a PCR amplification or freely present in samples of body fluids (blood, urine, sweat etc.)

The technique is sensitive and specific, due to the use of specific DNA(RNA) probes and easy to perform because of the quick DNA(RNA) Pt-labelling steps.

The universal labelling characteristics of the newly developed Pt label can be used in 3 ways to achieve a bound DNA(RNA) molecule.
1. It can be used to attach a detectable marker group to a polynucleotide sequence.
2. It can be used to attach polynucleotide sequences irreversibly to a solid phase (plastic, membranes, latex beads, hydrosols or microtiter plate wells).
3. A combination of 1 and 2

### ad 1:

In this example there is a twofold approach to the detection of biolytes biological analytes in test samples. Firstly a DNA probe can be labelled with the newly developed Pt labelling compound. This labelled probe can then be used to detect preformed hybrids on a membrane formed between the target DNA sequence and a primary probe. It is essential in this method that the primary probe recognizes a different sequence on the target than the secondary Pt labelled probe. In practice, this can be achieved for instance with RNA hybridization were a POLY A probe is used as a primary probe to immobilize all RNA (recognizable by its polyT tails) to a membrane.

The second approach differs slightly in that in this case the target can be labelled in the test sample fluid, because of the fast and very specific Pt labelling characteristics. A procedure like this would comprise a catch of the labelled target with an immobilized specific unlabelled DNA probe on a suitable membrane. Hence a dipstick version for DNA/RNA applications.

### ad 2:

To immobilize DNA probes or target DNA, a non-labelled Pt compound (that is a Pt compound with 2 free binding sites) can be used to act as a bridge between DNA and the surface of carriers (plastic, membrane, microtiter plates etc.)

It greatly enhances the usability of DNA sequences as catcher molecules in diagnostic assays, since there are little substances known to science that bind readily DNA in a spontaneous way. Introducing this Pt bridge molecule a wide field of new applications for the DNA technology has come within reach.

### ad 3: a combination of example 1 and 2

General: especially the use of the Pt linker molecule in latex or hydrosol assays is particularly interesting. The linker enables the coupling of DNA molecules to small particles. The DNA molecules can be hybridized to target material. A positive reaction is visualized by an agglutination of the particles, due to crosslinking of the DNA hybrid particle compounds.

A test like this can be made quantative, the rate of agglutination can be tuned and measured at a specific wavelength. Especially gold particles have the intrinsic characteristic that a shift in optimal wavelength occurs after agglutination.

### 3. Protein labelling using Pt-marker compounds in diagnostic test procedures.

The ability of Pt-marker compounds to interact with protein structures under specified conditions that differ from the DNA binding conditions, can be used to label e.g.:
a. mono or polyclonal antibodies to detect primary targets in test samples
b. protein derivatives used as antigens in test procedures based on the so called inhibition principle
c. specific protein structures that are known to interact specifically with other compounds, but generate a non immunogenic interaction with each other. e.g. streptavidin and biotin, and protein A and G with Immunoglobulines.

Labelled tracermolecules can be used to determine either qualitatively or quantitatively an amount of a certain biolyte in a suitable test format (dipsticks,elisa or others)

### 4. Long term acting proteins:

Of special interest is the potential use of the Pt linker to stabilise protein (biomolecules in general) in vivo. A therapeutical use of the Pt compound is that bio-substances used as therapeutics (insulin, factor VIII and the like) show a short term action once inside the body. The coupling of these substances to Pt compound may enhance the halflife of these molecules thus enabling longer acting substances to be produced and used in vivo. This implies less trouble for patients and more effective use of expensive drugs and medicines.

### 5. Detection of Platinated DNA probes with the the silver-enhancement technique.

Platinated DNA/RNA probe can be employed in hybridisation methods to detect DNA/RNA sequences in sample material. The introduction of a platinum compound at the site of the target enables the deposition of Ag molecules in a chemical reaction especially designed to reduce ionic Silver to metallic silver. At the site of a Pt nucleus a decomposition of metallic Silver(black) occurs due to the catalytic effect of the Pt nucleus.

Ionic silver is reduced by a reducing agent (e.g. Na-borohydrid, Hydrochinon) in solution. In a constant ratio the amount of silver deposited on the Pt is proportional to the length of the enhancement incubation.
Visualisation of a non-visible Pt nucleus can be accomplished by the empirical observation of the appearace of a black spot in the test sample.

The black spots indicate the site of specific probes binding and thus the site of specific target location. The technique enables a quick and easy diagnostic procedure for the detection of various microorganisms and gene translocations/abnormalities.

## Claims

1. A method for preparing a labelling substance of the formula: 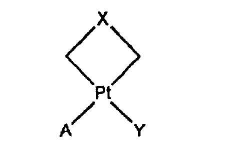 wherein X represents any stabilizing bridge, A represents a reactive moiety, and Y is a label, comprising the steps of:
- preparing a linker comprising a platinum compound of the formula: 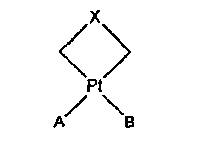
wherein A and B represent the same or different reactive moieties from a starting material comprising a compound of the structure: 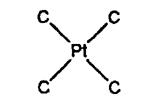 wherein C represents an electronegative moiety, and
- reacting the linker with a labelling group whereby Y is substituted for B.

2. A method according to claim 1, wherein A and B are the same.

3. A method according to claim 1 or 2, wherein X represents an aliphatic diamine.

4. A method according to claim 3, wherein X represents a diamine having 2-6 carbon atoms.

5. A method according to claim 2, wherein X is an ethylene diamine group.

6. A method according to claim 5, wherein A and B are selected from the group consisting of NO₃-, SO₃⁻, Cl-, I-, or other halogens.

7. A method according to any of the preceding claims, wherein C is a halogen, NO₃-, or SO₃-.

8. A method according to any of the preceding claims, wherein X is an ethylene diamine group, A and B represent NO₃-, and C represents a halogenic group, and wherein the starting material is reacted with ethylene diamine and the resulting compound is reacted with AgNO₃.

## Patentansprüche

1. Verfahren zur Herstellung einer Markierungssubstanz der Formel: 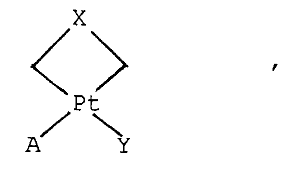 worin
X eine stabilisierende Brücke,
A eine reaktive Komponenete und
Y einen Marker darstellen,
mit folgenden Schritten:
- Herstellung eines Linkers, umfassend eine Platinverbindung der Formel: 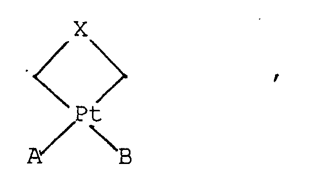
worin
A und B die gleiche oder unterschiedliche Reaktionskomponenten darstellen, und zwar ausgehend von einem Ausgangsmaterial mit einer Verbindung der Struktur: 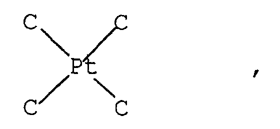 worin C eine elektronegative Komponente darstellt;
- Reagierenlassen des Linkers mit einer Markierungsgruppe, wobei B durch Y substituiert wird.

2. Verfahren nach Anspruch 1, worin A und B die gleichen Komponenten sind.

3. Verfahren nach Anspruch 1 oder 2, worin X ein aliphatisches Diamin darstellt.

4. Verfahren nach Anspruch 3, worin X ein Diamin mit 2 bis 6 Kohlenstoffatomen darstellt.

5. Verfahren nach Anspruch 2, worin X eine Ethylendiamingruppe darstellt.

6. Verfahren nach Anspruch 5, worin A und B aus der Gruppe bestehend aus NO₃-, SO₃-, Cl-, I- oder anderen Halogenen ausgewählt sind.

7. Verfahren nach einem der vorhergehenden Ansprüche,
worin C ein Halogen, NO₃- oder SO₃- ist.

8. Verfahren nach einem der vorhergehenden Ansprüche,
worin X eine Ethylendiamingruppe, A und B NO₃- und C eine Halogengruppe darstellen und
worin das Ausgangsmaterial mit Ethylendiamin zur Reaktion gebracht und die erhaltene Verbindung mit AgNO₃ reagieren gelassen wird.

## Revendications

1. Procédé de préparation d'une substance de marquage de formule: 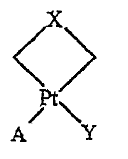 dans laquelle X représente un pont stabilisant quelconque, A représente un fragment réactif et Y est un marqueur, comprenant les étapes selon lesquelles:
- on prépare un élément de liaison comprenant un composé du platine de formule 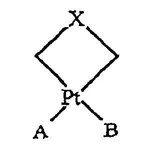
dans laquelle A et B représentent des fragments réactifs identiques ou différents, à partir d'un produit de départ comprenant un composé de structure 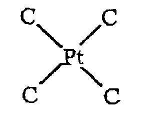 dans laquelle C représente un fragment électronégatif, et
- on fait réagir l'élément de liaison avec un groupe de marquage, grâce à quoi B est remplacé par Y.

2. Procédé selon la revendication 1, dans lequel A et B sont identiques.

3. Procédé selon la revendication 1 ou 2, dans lequel X représente une diamine aliphatique.

4. Procédé selon la revendication 3, dans lequel X représente une diamine de 2 à 6 atomes de carbone.

5. Procédé selon la revendication 2, dans lequel X est un groupe éthylènediamine.

6. Procédé selon la revendication 5, dans lequel A et B sont choisis dans le groupe constitué par NO₃⁻, SO₃⁻, Cl⁻, I⁻ ou d'autres halogènes.

7. Procédé selon l'une quelconque des revendications précédentes, dans lequel C est un halogène, NO₃⁻ ou SO₃⁻.

8. Procédé selon l'une quelconque des revendications précédentes, dans lequel X est un groupe éthylènediamine, A et B représentent NO₃⁻ et C représente un halogène, et dans lequel on fait réagir le produit de départ avec de l'éthylènediamine et on fait réagir le composé obtenu avec AgNO₃.
